(19) Europäisches Patentamt
European Patent Office
Office européen des brevets

(11) Publication number: 0 311 305 B1

# (12) EUROPEAN PATENT SPECIFICATION

(45) Date of publication of patent specification: **09.09.92** (51) Int. Cl.$^5$: **A61F 2/06**

(21) Application number: **88309052.4**

(22) Date of filing: **29.09.88**

## (54) Artificial blood vessel.

(30) Priority: **02.10.87 JP 247982/87**

(43) Date of publication of application:
**12.04.89 Bulletin 89/15**

(45) Publication of the grant of the patent:
**09.09.92 Bulletin 92/37**

(84) Designated Contracting States:
**CH DE FR GB IT LI NL**

(56) References cited:
**EP-A- 0 000 949**
**EP-A- 0 124 659**
**US-A- 4 319 363**
**US-A- 4 629 458**

**JPN. J. ARTIF. ORGANS, vol. 16, no. 3, 1987, pages 1527-1529; Y. NOISHIKI et al.: "Fundamental study of a growable graft"**

**Collagen Vol. III, p. 59-61, CRC Press Inc. Florida editor M.E. Nimni**

(73) Proprietor: **Koken Company Limited**
**5-18, Shimo-Ochiai 3-chome**
**Shinjuku-ku Tokyo(JP)**

(72) Inventor: **Noishiki, Yasuharu**
**827 Yamada Misasa-cho,Takaku-gun**
**Tottori-Ken, 682-02(JP)**
Inventor: **Yamane, Yoshihisa**
**214-1-Yatsuya, Kurayoshi-shi**
**Tottori-ken,682(JP)**
Inventor: **Miyata, Teruo**
**314, 3-6-29, Shimoochiai, Shinjuku-ku**
**Tokyo, 161(JP)**

(74) Representative: **Gore, Peter Manson et al**
**W.P. THOMPSON & CO. Coopers Building**
**Church Street**
**Liverpool L1 3AB(GB)**

EP 0 311 305 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to an artificial blood vessel, particularly one capable of growing in consonance with the growth of a patient, for example, a child.

Conventional artificial blood vessels retain their original dimensions, such as, for example, thickness and length, after implantation operations. The conventional artificial vessels do not provide any inconveniences with respect to their original dimensions when they are used in adults. However, problems occur when these blood vessels are implanted into children or young animals, who are still experiencing growth. One problem is that after a conventional artificial blood vessel is implanted, relative stenosis occurs with the growth of the patients. In other words, as the child or young animal grows, its natural blood vessels grow. However, the implanted artificial blood vessel does not grow. This creates relative stenosis in that the artificial blood vessel in narrowed or has a smaller diameter than the natural blood vessels of the host child or young animal. For this reason, at least second and even third operations are required, in which thicker blood vessels are implanted into the host which are consonant with the growth of the host patient. Such further operations become a burned for the patients and is also accompanied with the danger associated with repeated operations. For these reasons, there is a great need for artificial blood vessels capable of growing in consonance with the growth of the body.

The inventors of the present application have previously proposed a blood vessel comprising a polyester mesh tube having its lumen (inner diameter) reduced by tying each mesh with an absorbent thread. This tying makes the mesh size variable. A silicone tube is snugly inserted into the lumen within the lumen (inner diameter) of the polyester mesh tube having its inner diameter reduced by tying. This combined tube is implanted subcutaneously in a dog. After about 20 days, the combined tube is removed together with the connective tissue adhering to the mesh tube. The silicone tube in the lumen (inner diameter) is then removed. The resulting tube comprising the polyester mesh tube has become intertwined with connective tissue and has its thickness increased with the connective tissue of the dog and is useful as an artificial blood vessel. When this blood vessel is implanted into a host, the connective tissue is completely absorbed by the host. New tissues of the host itself form a new blood vessel wall. The absorbent thread tying the polyester mesh tube is also absorbed. Thereafter, it is expected that the polyester mesh returns to its original size. With the expansion of the polyester mesh, the new wall of blood vessel becomes thicker. In this way, the artificial blood vessel grows. This matter was published by the inventors of the present application in JINKO-ZOKI (Artificial Organs), 16,3, pp.1527-1529, (1987).

US-A-4 629 458 describes a tubular graft made of collagenous tissue with a reinforcing structure surrounding the lumen. This structure is a coiled monofilament which is embedded within or positioned about collagen.

Further study of artificial blood vessels by the inventors of the present application has led to the present invention. It has now been found possible to provide an artificial blood vessel capable of growing or thickening in proportion to the growth of a child or young animal patient.

According to the present invention, there is provided an artificial blood vessel which in use is capable of growing in consonance with the growth of a patient, characterised by a crosslinked collagen inner tube wherein the rate of crosslinking, as measured by the rate of reaction of $\epsilon$-amino groups on the side chains in collagen is from about 1% to about 50%, and generally spaced therefrom an outer mesh tube.

The artificial blood vessel of the present invention does not deteriorate in the host's body after implantation. The artificial blood vessel of the present invention can be implanted into any animal which experiences growth or thickening of blood vessels from birth to adulthood. Preferred host animals are mammals and man.

Any kind of collagen tube can be used in the present invention. For example, a collagen tube can be made from purified collagen which is pepsin solubilized (atelocollagen) and/or smashed insoluble tendon or corium. With solubilized collagen, the collagen tube can be prepared, for example, by extruding collagen solution through a double-walled nozzle into a coagulating bath. A representative example of preparing such a collagen tube is to disperse 4% insoluble corium collagen in 1% atelocollagen solution pH 3.0. This solution is degassed and extruded into a coagulation bath which is a saturated NaCl solution through a double-walled nozzle having an inner diameter of 4mm and an outer diameter of 8mm. Afterwards, the coagulated tube is neutralized with 1% $HN_3OH$ and washed with water.

Alternatively, animal tube-like vessels, for example, blood vessels or urethra of animals can be used for the collagen tube. The animals include animals other than man, such as, for example, blood vessels from bovine, porcine, ovine, dog and fowl. As for man, blood vessels, which are removed during an operation for varicosis, can be stored and later used as the collagen tube. When tube-like vessels of animals are used, such as, for example, blood vessels of humans, the blood vessels are treated to detach cells, such as, for

example, endothelial cells and muscle cells, from the blood vessels. This removal can be done by ultrasonic treatment or by shaking the tube-like tissue in 0.5% trypsin for 1 hour.

After an appropriate collagen tube is obtained, the collagen tube is crosslinked. Crosslinking agents which may be used in the present invention are preferably hydrophilic crosslinking agents. By using hydrophilic crosslinking agents, the resulting artificial blood vessels can be rendered highly hydrophilic and an antithrombogenicity property can be imparted thereto. Examples of crosslinking agents include glutaraldehyde, hexamethylene diisocyanate, and polyepoxy crosslinking agents. Representative polyepoxy crosslinking agents include polyglycidyl ethers of glycerols (having a polymerization degree of 1 to 3), polyglycidyl ethers of polyols, and, in particular, glycerol diglycidyl ether, glycerol triglycidyl ether, diglycerol tetraglycidyl ether, ethyleneglycol glycidyl ether, and polyethylene glycol diglycidyl ether having a polymerization degree of 1 to 22.

The rate of crosslinking which is measured as the rate of reaction of the $\epsilon$-amino groups on the side chains in collagen, and normally ranges from about 1% to 50% or less, more preferably up to 30%. By limiting the rate of crosslinking to such low levels, the implanted artificial blood vessel can gradually increase in diameter up to the diameter of the outer mesh tube. The rate of growth of the artificial blood vessel when implanted in a child or young animal can be controlled by the amount of crosslinking of the collagen tube. A higher amount of crosslinking of the collagen tube will result in slower blood vessel growth, and a lower amount of crosslinking of the collagen tube will result in faster blood vessel growth. Thus, thick and stable blood vessels can be obtained with the present invention which are identical or nearly identical in size to the blood vessels of the host child or young animal and which grow or thicken in an amount proportional to the growth of the host child or young animal.

The diameter of the mesh tube is generally selected to match an expected diameter of a growing child at the time of implantation or at some future time of growth. The diameter of the mesh tube can be selected at an appropriate diameter, so that the artificial blood vessel will stop growing at a particular diameter. This control can be important for limiting the growth of the artificial blood vessel for safety. Mesh tubes used for covering the outer surface of collagen tubes are preferably polyester mesh tubes having the mesh size of 0.01mm to 2mm. However, the other sizes and materials for the mesh tubes can be used in the present invention. Any mesh material can be used, as long as it is inert to the host. Representative meshes which can be used in the present invention include polyester, nylon, polypropylene chloride, polyvinylidene chloride, and polyacryl meshes. The mesh size or mesh opening can vary. For example, the mesh tube can have a mesh size or mesh opening of 0.01mm to 2mm. The mesh tube may or may not be secured to collagen tube. When secured, either an inert or absorbent thread can be used to tie the polyester mesh to the collagen tube. When the artificial blood vessel is implanted in the host, the collagen tube can be secured to the host by either an inert or absorbent thread, and the polyester tube can be secured by an absorbent thread or a non-absorbent, inert thread. The joining of the collagen tube and the polyester mesh of the artificial blood vessel of the present invention to the tissue of the host is usually done by the use of sutures. The absorbent threads are later absorbed by the host after a period of time.

When the artificial blood vessel of the present invention is implanted in a child or young animal, the artificial blood vessel grows and increases in thickness to match that of the child's natural blood vessels. The growth and increase in size of the blood vessel is due to new growth by the child or young animal inside of and onto the polyester mesh. This new growth of the child or young animal encompasses and surrounds the collagen tube, which is later absorbed by the child. In other words, a new blood vessel wall is grown by the host. The growth of the artificial blood vessel or the thickening thereof continues as the child ages. For example after implantation, the artificial blood vessel reaches a thickness matching that of the child. Thereafter, the artificial blood vessel increases in thickness as the child's natural blood vessels increase in thickness, namely, matching the growth in thickness of the child's natural blood vessels.

The present invention will now be further described with reference to, but is not limited to, the following Example.

## EXAMPLE

A large, human saphenous vein, which was taken during an operation for varicosis and stored in 70% ethanol, was immersed in distilled water and subjected to ultrasonic treatment to detach endothelial cells, muscle cells, and other undesired material in the vein. The resulting tube composed mainly of collagen was crosslinked with Denacol EX-861, (a tradename of Nagase Kasie Kogyo K.K.) Denacol EX-861 is a hydrophilic crosslinking agent and contains polyethylene glycol diglycidyl ether having a polymerization degree of 22. Polyethylene glycol diglycidyl ether can be represented by the following formula:

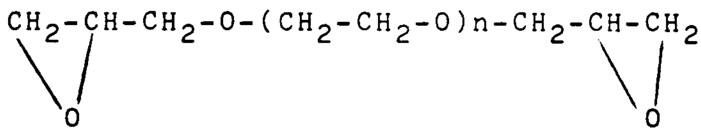

where n is 1 to 22.

Thereafter, the crosslinked collagen was thoroughly washed with water, and stored in 70% ethanol.

The collagen tube thus obtained (inner diameter 4mm, length 6cm) was covered on its outer surface with a polyester mesh (1mm mesh opening) tube having rough meshes. The polyester mesh has an inner diameter of 10mm and a length of 6cm. The artificial blood vessel of the combined collagen tube within the polyester mesh was implanted in thoracis aorta descendants in each of four young dogs (body weight is 5 to 7kg), in such a way that the ends of the artery wall of the host dog extending the anastomosed portions was secured by suture to the artificial blood vessel. Six months after the implantation, the experimental animals were subject to an aortogram to measure the diameter of the implanted artificial blood vessels. Also samples of the blood vessels were taken periodically and observed microscopically and with a light microscope. The body weights of the dogs were 8.5 to 10.3kg at the time of aortogram and, thus, the dogs were about 49% heavier than at the time of implantation. As this time, the diameter of the blood vessels was 9mm, while the anastomosed portions did not grow and had a diameter of 5mm. This is because the anastomosed portions were made by continuous anastomose. The artificial blood vessels, except the anastomosed portions had grown uniformly, to have the same thickness as that of the host dog's blood vessels. No formation of aneurysm was observed. Histologic inspection indicated that the tissue originating from the large saphenous vein adhered to, such as being forced against, the inner surface of the polyester mesh tube and formed a new wall of blood vessel together with newly formed membrane on the outer surface of the mesh tube.

As described above, the blood vessel of the present invention grows in consonance with the growth of a host as demonstrated, in particular, by the Example. When the blood vessel according to the present invention is implanted in a human child or young animal, the need for the conventional second and third operations and the danger associated therewith can be avoided. In addition, because of the incorporation of a polyester mesh which does not undergo deterioration in a living body, the growth of the blood vessel can be controlled and stopped at a desired thickness for safety.

## Claims

1. An artificial blood vessel, which in use is capable of growing in consonance with the growth of a patient, characterised by a crosslinked collagen inner tube wherein the rate of crosslinking, as measured by the rate of reaction of $\epsilon$-amino groups on the side chains in collagen is from about 1% to about 50%, and generally spaced therefrom an outer mesh tube.

2. An artificial blood vessel as claimed in claim 1, in which the outer mesh tube has an inner diameter at least 2mm greater than an outer diameter of the collagen tube.

3. An artificial blood vessel according to claim 1 or 2, wherein the collagen tube is a tube prepared by extruding a collagen solution through a double-walled nozzle into a coagulating solution.

4. An artificial blood vessel according to claim 1, 2 or 3, wherein the collagen tube is a blood vessel of an animal or a blood vessel of a human obtained during an operation for varicosis.

5. An artificial blood vessel according to any of claims 1 to 4, wherein the collagen tube is crosslinked with a hydrophilic crosslinking agent selected from glutaraldehyde, hexamethylene diisocyanate, and polyepoxy.

6. An artificial blood vessel according to claim 5, wherein the polyepoxy crosslinking agent is selected from glycerol diglycidyl ether, glycol triglycidyl ether, diglycerol tetraglycidyl ether, ethyleneglycol glycidyl ether, and polyethylene glycol diglycidyl ether having a polymerization degree of 1 to 22.

7. An artificial blood vessel according to any of claims 1 to 6, wherein the mesh tube is a polyester mesh tube and has a mesh size of 0.01mm to 2mm.

8. An artificial blood vessel according to any of claims 1 to 7, wherein in use the collagen tube is absorbed and replaced with a new blood vessel made of tissue of the patient.

9. An artificial blood vessel according to claim 8, wherein the new blood vessel has a thickness matching that of corresponding natural blood vessels of the patient.

10. An artificial blood vessel according to claim 9, wherein over a period of time the new blood vessel increases in thickness corresponding to an increase of the natural blood vessels.

**Patentansprüche**

1. Künstliches Blutgefäß, das im Gebrauch fähig ist, in Einklang mit dem Wachstum eines Patienten zu wachsen, gekennzeichnet durch ein Innenrohr aus vernetztem Collagen, worin der vernetzungsgrad, wie durch den Umsetzungsgrad von $\epsilon$-Aminogruppen an den Seitenketten im Collagen gemessen, von ungefähr 1 % bis ungefähr 50 % beträgt und ein im allgemeinen in einem Abstand davon angeordnetes äußeres Gitterrohr.

2. Künstliches Blutgefäß nach Anspruch 1, in dem das äußere Gitterrohr einen Innendurchmesser von mindestens 2 mm mehr als ein Außendurchmesser des Collagenrohrs aufweist.

3. Künstliches Blutgefäß nach Anspruch 1 oder 2, worin das Collagenrohr ein durch Extrudieren einer Collagenlösung durch eine doppelwandige Düse in eine Koagulationslösung hergestelltes Rohr ist.

4. Künstliches Blutgefäß nach Anspruch 1, 2 oder 3, worin das Collagenrohr ein Blutgefäß eines Tieres oder ein Blutgefäß eines Menschen ist, das bei einer Operation wegen Varikose erhalten wurde.

5. Künstliches Blutgefäß nach einem der Ansprüche 1 bis 4, worin das Collagenrohr mit einem hydrophilen Vernetzungsmittel ausgewählt aus Glutaraldehyd, Hexamethylendiisocyanat und Polyepoxy vernetzt ist.

6. Künstliches Blutgefäß nach Anspruch 5, worin das Polyepoxy-Vernetzungsmittel ausgewählt ist aus Glycerindiglycidylether, Glycoltriglycidylether, Diglycerintetraglycidylether, Ethylenglycolglycidylether und Polyethylenglycoldiglycidylether mit einem Polymerisationsgrad von 1 bis 22.

7. Künstliches Blutgefäß nach einem der Ansprüche 1 bis 6, worin das Gitterrohr ein Polyestergitterrohr ist und eine Maschenweite von 0,01 mm bis 2 mm aufweist.

8. Künstliches Blutgefäß nach einem der Ansprüche 1 bis 7, worin das Collagenrohr im Gebrauch absorbiert und ersetzt wird mit einem neuen Blutgefäß, das aus Gewebe des Patienten hergestellt ist.

9. Künstliches Blutgefäß nach Anspruch 8, worin das neue Blutgefäß eine Dicke aufweist, die zu der von entsprechenden natürlichen Blutgefäßen des Patienten passend ist.

10. Künstliches Blutgefäß nach Anspruch 9, worin über einen Zeitraum das neue Blutgefäß an Dicke zunimmt, entsprechend einer Zunahme der natürlichen Blutgefäße.

**Revendications**

1. Vaisseau sanguin artificiel qui est capable en service de se développer en conformité avec le développement d'un patient, caractérisé par un tube interne de collagène réticulé dont le taux de réticulation, tel que mesuré par le taux de réaction des groupes $\epsilon$-amino sur les chaînes latérales du collagène, est d'environ 1 % à environ 50 %, et un tube externe à mailles qui en est globalement espacé.

2. Vaisseau sanguin artificiel selon la revendication 1, dans lequel le tube externe à mailles a un diamètre

interne supérieur d'au moins 2 mm au diamètre externe du tube de collagène.

3. Vaisseau sanguin artificiel selon la revendication 1 ou 2, dans lequel le tube de collagène est un tube préparé en extrudant une solution de collagène par une buse à double paroi dans une solution de coagulation.

4. Vaisseau sanguin artificiel selon la revendication 1, 2 ou 3, dans lequel le tube de collagène est un vaisseau sanguin d'un animal ou un vaisseau sanguin d'un être humain obtenu pendant une opération sur des varices.

5. Vaisseau sanguin artificiel selon l'une quelconque des revendications 1 à 4, dans lequel le tube de collagène est réticulé par un agent de réticulation hydrophile choisi parmi le glutaraldéhyde, le diisocyanate d'hexaméthylène et un polyépoxyde.

6. Vaisseau sanguin artificiel selon la revendication 5, dans lequel l'agent de réticulation de type polyépoxyde est choisi parmi l'éther diglycidylique de glycérol, l'éther triglycidylique de glycol, l'éther tétraglycidylique de diglycérol, l'éther glycidylique d'éthylène-glycol et un éther diglycidylique de polyéthylène-glycol ayant un degré de polymérisation de 1 à 22.

7. Vaisseau sanguin artificiel selon l'une quelconque des revendications 1 à 6, dans lequel le tube à mailles est un tube à mailles en polyester et a une ouverture de mailles de 0,01 mm à 2 mm.

8. Vaisseau sanguin artificiel selon l'une quelconque des revendications 1 à 7, dans lequel, en service, le tube de collagène est absorbé et remplacé par un nouveau vaisseau sanguin constitué du tissu du patient.

9. Vaisseau sanguin artificiel selon la revendication 8, dans lequel le nouveau vaisseau sanguin a une épaisseur coïncidant avec celle des vaisseaux sanguins naturels correspondants du patient.

10. Vaisseau sanguin artificiel selon la revendication 9, dans lequel, en une certaine période de temps, l'épaisseur du nouveau vaisseau sanguin augmente corrélativement à une augmentation des vaisseaux sanguins naturels.